# EUROPEAN PATENT SPECIFICATION

(11) **EP 2 616 020 B1**
(45) Date of publication and mention of the grant of the patent: **16.03.2022**
(21) Application number: 11807999.5
(22) Date of filing: 12.09.2011
(51) Int. Cl.: A61F 2/915

(54) **STENTS WITH LOW STRUT THICKNESS AND VARIABLE STRUT GEOMETRY**
STENTS MIT GERINGER STREBENDICKE UND VERÄNDERLICHER STREBENGEOMETRIE
STENTS PRÉSENTANT UNE FAIBLE ÉPAISSEUR D'ENTRETOISE ET UNE GÉOMÉTRIE D'ENTRETOISE VARIABLE

(30) Priority: 13.09.2010 IN MU25232010
(43) Date of publication of application: 24.07.2013
(73) Proprietor: Meril Life Sciences Pvt Ltd., Vapi 396 191 GUJ (IN)
(72) Inventor: VYAS, Rajnikant Gandalal, Gujarat(GJ) (IN); THAKOR, Utpal Devendra, Gujarat(GJ) (IN)
(74) Representative: Forrest, Stuart
(86) International application number: PCT/IN2011/000622
(87) International publication number: WO 2012/035550

(56) References cited:
- EP-A2- 1 674 118
- WO-A1-99/17680
- WO-A1-02/051335
- WO-A2-03/105695
- US-B1- 6 540 775
- US-B1- 6 896 697

## Description

### TECHNICAL FIELD:

This invention relates to balloon expandable stents, which are capable of being implanted into a mammalian body lumen, such as a blood vessel.

### BACKGROUND OF THE INVENTION:

Stents are used to treat atherosclerotic stenosis or other type of blockages in body lumen like blood vessels or to expand the lumen that has narrowed due to disease. The function of the stent is to expand the lumen diameter by pressing the plaque to the vessel wall and to maintain patency of the lumen of the blood vessel thereafter at the location of its implantation. The stent may be bare metal or may be coated with therapeutic agent/s and/or biocompatible material/s for beneficial effects like reduction in inflammation, minimize restenosis etc.

Stents are cylindrical in shape with a scaffold structure. Such structures are formed on a metal tube by cutting the tube using a laser beam. The metal tubes are made of biocompatible metals like stainless steel, cobalt chromium alloys, tantalum, platinum etc. The laser cut tube is then cleaned, heat treated and electropoilished. The finished stent is then mounted on a balloon catheter by crimping process such that it holds tightly over the balloon and attains a considerably lower diameter. The crimped stent mounted on catheter is directed to the site of the disease (blockage or narrowed lumen). At the site of the disease, the balloon is inflated by application of hydraulic pressure to expand the stent radially to desired diameter. Radial expansion of the stent presses the plaque to the wall of the vessel by which the restriction to the flow of blood in the vessel is removed. The balloon is then deflated by removing the hydraulic pressure and withdrawn from the body of the patient. On expansion, the stent material attains plastic deformation and hence the stent does not recoil back to its original shape and remains in expanded state keeping the lumen patent. To withstand forces exerted by the blood vessel, the stent scaffold structure should have sufficient radial strength and fatigue strength. The scaffold structure should also be dense enough to prevent prolapse of the plaque. The stent should exhibit required radiopacity for ease of implantation.

The stents are also made from wires arranged in a variety of patterns, helically wound coiled springs etc. which can be expanded using a balloon catheter in the body lumen. The stents are also made from biocompatible and biodegradable polymeric materials by methods known in art. Superelastic materials like Nitinol alloys are also used for making self expanding stents. Such stents do not require balloon catheters and are implanted by removal of the restraining sheath whereby the stent expands on its own in the lumen.

Stents have been used effectively for quite a long time and its safety and efficacy are well established. The main issues of a stent are restenosis and in-stent thrombosis. One of the important causes of these adverse effects is injury of the artery caused by implantation of the stent. The injury leads to restenosis and delayed endothelialization. These adverse effects can be reduced if injury to the artery is reduced.

The stents exhibit characteristic "dog boning" effect during implantation of the stent. Dog boning is the result of non-uniform radial expansion of the stent along its longitudinal axis. The proximal and distal ends of the stent expand before expansion of the central portion giving it a shape like a "dog bone". The ends of the stent thus attain lumen diameter earlier than the central portion. This happens because the central potion of the stent offers greater radial resistance than the end portions which do not have structural support at their tips. This causes the proximal and distal ends of the stent to over-expand making the end portions to protrude radially outwards. This causes the stent ends to slip relative to the balloon toward the central portion causing reduction in overall length of the stent. The central section continues to expand to attain final diameter which drives the stent ends radially upwards and axially outwards. This causes the ends to enter deeper into the lumen wall causing injury to the wall of the lumen. The stent deployment may also be affected adversely due to this phenomenon. This type of injury can be reduced considerably or eliminated if the stent scaffold is designed to eliminate dog-boning effect.

It is well established that the thickness of the struts of a stent plays an important role in injuring of the artery. Thinner struts cause less injury compared to thicker struts. Thus, the injury of the artery can be reduced by making the struts as thin as practically possible. While deciding the thickness of the struts, care should be taken so that important mechanical properties of the stent like radial strength and fatigue resistance are adequate to withstand arterial forces.

The problem of "dog boning" can be eliminated by making the central portion of the stent expand comparatively earlier than the end portions. In such a case the central portion of the stent will attain lumen diameter (or contact the wall of the lumen) earlier than the end portions. The area of artery covered by the central portion of the stent expended earlier is generally large. Thus the expansion force applied to the stent is distributed over larger lumen area which causes considerably less injury compared to the injury caused by the dog boning effect.

The early expansion of the ends of the stent can be prevented by making its end portions structurally stronger in relation to its central portion. As the central portion of the stent is mechanically weaker compared to end portions, it will expand earlier than the end portions of the stent. The differential strengths of the stent sections can be achieved by varying the scaffold geometry of the struts of the stent across its length. Care should be taken to configure the scaffold structure in such a way that at desired expansion pressure, the entire stent attains specified diameter, end as well as the central portion.

The injury to the artery wall can be minimized further by reducing the thickness of the struts of the stent scaffold structure. It is well established that the stent with less strut thickness causes less injury compared to the stent with thicker struts. Kastrati A, Schömig A, Dirschinger J, et al. In their paper "Strut Thickness Effect on Restenosis Outcome (ISAR STEREO Trial)" published in Circulation 2001; 103:2816-2821 discussed this subject in detail. The incidence of angiographic restenosis was 15.0% in the group of patients treated with stents of thin struts (50 microns thickness) against restenosis of 25.8% in the group treated with stents with thicker struts (140 microns thickness). Clinical restenosis was also significantly reduced, with a reintervention rate of 8.6% among thin-strut patients and 13.8% among thick-strut patients.

These findings were reconfirmed by Kastrati A, et al in their paper "Strut Thickness Effect on Restenosis Outcome (ISAR STEREO-2 Trial)" published in J. Am. Coll. Cardiol, 2003; 41:1283-8. The incidence of angiographic restenosis was 17.9% in the group of patients treated with stents of thin struts (50 microns thickness) against restenosis of 31.4% in the group treated with stents with thicker struts (140 microns thickness). Target Vessel Revascularization (TVR) due to restenosis was required in 12.3% of the patients in thin strut group against 21.9% required in patients of the thick strut group.

In conclusion from both the above studies it was established that the use of thinner strut device is associated with a significant reduction in angiographic and clinical restenosis after coronary stenting.

The coronary stents now available in the market have strut thickness varying from 140 microns (Cypher^{®} of Cordis) to 60 microns (PRO-Kinetic^{®} of Biotronik). General trend is to make the new generation stents with thinner struts.

While the elimination of dog-boning effect and making thin struts are desirable for better clinical performance of a stent, it is extremely important that other properties of stent like radial strength, fatigue resistance, trackability, pushability, foreshortening, elastic recoil etc. are not affected adversely. The reduction in strut thickness reduces the radial strength of the stent. The reduction in the thickness of the struts must be compensated in some way to impart required radial and fatigue strengths to the stent. In addition, the stent should have adequate radiopacity for ease of implantation. Hence, it is extremely important to strike balance between the mass of the stent and its radiopacity. The stent scaffold geometry should provide enough support to the compressed plaque to avoid its prolapse and also should exhibit required crossing profile. The scaffold geometry of the struts should thus be designed in such a way that the dog boning is eliminated and struts are made thinner without affecting these properties adversely.

US patent No. 6,273,910 describes different configurations of a stent where geometry of scaffold struts is varied axially to achieve different mechanical strengths in the central and end portions of the stent. This prior art achieves this by two alternate structural principles. The first principle is based on the fact that the structures formed with shorter axial lengths, shorter circumferential dimensions, or wider cross-sections are more resistant to circumferential deformation than structures having respectively longer axial lengths, larger circumferential dimensions, or more narrow cross-sections. Accordingly, structures having greater resistance to circumferential deformation are utilized to form cylindrical elements having greater resistance to radial expansion viz. end portions. The second principle is based on the fact that the structures having wider cross sections are more resistant to circumferential deformation than structures having narrower cross sections. Therefore, end portions have scaffold structures with wider cross section than the central section. This prior art also mentions about achieving differential strengths by using materials with different strengths to build different portions of the stent. However, it is not simple to make a stent using different materials for different portions of the same stent.

US patent 7,044,963 discusses similar principles for achieving differential strengths across the axis of a stent. This prior art achieves differential strengths by (a) increasing or decreasing thickness or width of elements of one or more sections relative to other sections (b) increasing or decreasing axial length (c) changing cell shape and size (say connectors are changed from U to S or Z shape) (d) change material properties by either changing materials or by differential heat treatment. These are general structural principles of changing mechanical properties and are well known scientific principles. However, it is extremely difficult to use different materials for different sections of the same stent or apply different heat treatment processes to different parts of the same stent.

Publication No. WO 01/34241 A1 describes stents which are used preferentially for cerebral vasculature. The emphasis is placed on reducing radial strength to expand the stent at a lower pressure compared to coronary stent. This stent has circumferential U shaped members with straight links which connect these members. The number and pattern of providing these connecting links are varied to achieve different stent characteristics (strength, flexibility etc). Connecting links of variable width and thickness are provided for varying characteristics; however, no method to get varying thickness along longitudinal axis is specified. This stent expands uniformly at 6 - 8 atm pressure. US 6,896,697 discloses an intravascular stent assembly for implantation in a body vessel, such as a coronary artery. The stent includes undulating circumferential rings having peaks on the proximal end and valleys on the distal end. Adjacent rings are coupled together between peaks on one ring and valleys on the proximally adjacent ring. To increase flexibility of the stent, the links do not couple all of the peaks on a ring to the proximally adjacent ring.

WO 03/105695 discloses a deformable medical implant, comprising: a body defining at least two anchor points, wherein the body is adapted to be deformed so that the two anchor points are moved relative to each other; at least two elongate extensions, each extension fixed to one anchor point; a bridge coupling at least two of said extensions to each other; and at least two hinges defined on at least one of said extensions, two of said at least two hinges having different preferred bending directions and being defined on one extension.

WO 99/17680 represents the most relevant prior art for the subject-matter of claim 1. it discloses prostheses and methods for their endolumenal placement within body lumens particularly blood vessels. The prosthesis of the comprises a plurality of radially expansible rings having a plurality of beams connecting axially remote points on adjacent rings, and a plurality of expansion joints connecting axially proximate points on adjacent rings. The beams maintain the remote points at a fixed distance when the prosthesis is radially expanded and the expansion joints allow for relative movement of the proximate points during delivery of the prothesis. The combination of the beams and expansion joints minimizes the risk of injury to the body lumen during delivery of the prosthesis to a target site and provides improved tracking capabilities.

WO 02/051335 discloses implantable intravascular stent comprising a plurality of expandable stent modules. Each of the modules preferably comprises first and second annular elements each comprising thin filaments formed in an undulating pattern consisting of alternating peaks and troughs. A plurality of first connectors secure the first and annular second elements together in radial alignment to define a cylindrical opening. A plurality of second connectors are also provided for joining adjacent pairs of stent modules together at radially spaced locations. The second connectors are extensible between contracted and extended configurations to allow the stent to conform to curves in the vessel wall during deployment of the stent and to prevent foreshortening. The second connectors also inhibit prolapse of atherosclerotic plaque or vascular tissue at locations between the module

EP 1 674 118 discloses a biocompatible material which may be configured into any number of implantable medical devices including intraluminal stents. The biocompatible material may comprise metallic and non-metallic materials. These materials may be designed with a microstructure that facilitates or enables the design of devices with a wide range of geometries adaptable to various loading conditions. In one embodiment an intraluminal scaffold is described. It comprises at least one flexible connector element having a luminal surface and an abluminal surface, the flexible connector element having a predetermined wall thickness, wherein the wall thickness is defined by the radial distance between the luminal surface and the abluminal surface, and a predetermined feature width, wherein an area bounded by the wall thickness and the feature width comprises a plurality of zones undergoing at least one of tensile, compressive or substantially zero stress change due to external loading, the flexible connector element being fabricated from a metallic material processed to have a microstructure with a granularity of about 32 microns or less and at least one internal grain boundary within the bounded area

US 6,540,775 discloses an open cell stent that has adjacent sets of circumferential struts connected by means of highly flexible, undulating, connecting struts. To decrease outward flaring of the circumferential struts when the pre-deployed stent is advanced through highly curved vessels, each unconnected strut of the sets of circumferential struts has a decreased longitudinal length as compared to the longitudinal length of the circumferential struts that are connected by the flexible connecting struts. To decrease the propensity for outward flaring of the end set of circumferential struts, the longitudinal length of the end set of circumferential struts is shorter than the longitudinal length of the interior set of circumferential struts. Also, the attachment point of the flexible connecting struts is off the center of the curved sections of the circumferential struts, thus also decreasing the tendency for outward flaring of the end set of circumferential struts.

A simple way to achieve differential strength is to change strut geometry across the axis of the stent by incorporating such changes in a simple and practical manner.

### SUMMARY OF THE INVENTION:

The present invention describes a balloon expandable stent made from a biocompatible Cobalt-Chromium alloy, for implantation in a mammalian body lumen such as a blood vessel like an artery. The stent may be used as platform for coating with therapeutic agent/s and/or with biocompatible material/s for beneficial clinical effects.

According to the present invention, the stent consists of a balloon expandable metallic stent according to claim 1.

The scaffold structure of the end sections is designed to achieve higher mechanical strength compared to that of the central section. Since both the end sections of the stent are designed to offer greater resistance to radial expansion compared to the central section, the central section of the stent will expand earlier than the end sections during deployment of the stent in the body lumen by a balloon catheter. Thus the central section of the stent will contact the wall of the lumen earlier than the end sections eliminating the dog-boning effect. This differential strength is achieved without affecting other properties of the stent adversely.

Geometry of the scaffold structure can be varied in many ways to achieve different mechanical strengths i.e. resistance to expansion. As described in the prior art, this can be done by varying the axial lengths, circumferential dimensions or width of cross-sections, varying the width of the cross section of the struts, varying the thickness of the struts and using different materials across the axis of the stent and even by applying different heat treatment to different parts of the stent. These methods have several disadvantages. Very wide cross section will lead to a relatively open structure which can not contain the plaque effectively leading to tissue prolapse resulting into restenosis or embolization. Another disadvantage is different crossing profiles in deferent sections of the stent. Using different materials in the same stent makes its construction quite difficult and tedious. In addition, it may lead to galvanic corrosion. Changing thickness of a stent across its axis requires special methods to polish the stent differentially. Increasing the width of the struts is not very effective in increasing the strength as the section modulus is directly proportional to its width and the width of the stent strut can not be increased beyond a limit where the strut will start touching each other during crimping on the balloon catheter. It is tedious and difficult to apply differential heat treatment to different parts of the stent across its axis.

It is simple to alter the geometry of a stent across its axis. If this is done ingeniously, the geometry will not vary too widely across the axis of the stent and still impart adequate differential structural strengths to achieve desired results. There is no need either to vary thickness of the stent across its length or not necessary to apply differential heat treatment or use different materials in the same stent. This invention is based around this theme and sought protection for the same.

The scaffold structure of a stent has repetitive radially expandable rows of geometrical shapes across its circumference which may be termed as cylindrical elements forming rings. The shape of an element and the way such elements are interconnected with each other can be manipulated to achieve different structural properties i.e. mechanical strengths which lead to different resistance to expansion. There exists large design flexibility in creating different shapes. This flexibility should be used keeping in mind other desirable properties of the stent. The stent structure is formed by placement of these elements in a specific pattern to form specific shapes and interconnected array of struts. The elements in the pattern should be close enough such that on expansion of the stent, the plaque or dissections of the body lumen are effectively pressed back in position against the wall of the lumen giving adequate support to prevent tissue prolapse. At the same time, these elements should not be so close as to affect flexibility adversely, interfere with each other during crimping of the stent on the balloon of a catheter or exhibit inadequate crossing profile. They should be stiff enough to impart required radial and fatigue resistance strength to the stent. The elements should undergo enough plastic deformation on expansion at specified pressure such that the elastic recoil is within acceptable limits. When the stent is expanded radially, its diameter increases which causes reduction in its length. The shape and arrangement of the elements should compensate this reduction in stent length to bring foreshortening of the expanded stent within acceptable limits. This is achieved by causing the specific strut elements to elongate in unison with radial expansion. The elements should have sufficient mass to exhibit enough radiopacity for ease of implantation procedure. Though different sections of the stent have different mechanical strength across its axis, the stent should achieve its specified diameter uniformly across its entire length when the rated deployment pressure is applied to the balloon catheter.

The structural elements in the stent described in this invention are configured to give open or closed shaped structures which may be termed as 'cell' or 'cell structure'. Using struts having same width and thickness for making an element, the resistance to expansion offered by an open cell structure is lower compared to that offered by a closed cell structure. This means that closed cells will offer higher resistance to expansion compared to open cells. Additional design flexibility is achieved by making these cells with varying lengths and widths of the elements. Structural elements with larger length will result in cell with higher width and give lower strength. On the other hand, structural elements with shorter length will result in cell with lower width and give higher strength. Higher width of the struts will have higher strength and offer more resistance to expansion compared to cells with elements of less width and vice versa.

The end section of a stent is defined as the section at either proximal or distal end of the stent. This section ends when the closed cell configuration changes to open cell configuration. Similarly the central section is defined as the section of the stent sandwiched between two end sections and it consists of open cell configuration. The central section ends when open cell configuration changes to the closed configuration. The stent structure of the present invention is described in following sections. The terms 'element' and 'strut' are used interchangeably throughout the specifications.

The scaffold structure of the stent of present invention generally consists of sinusoidal wave type elements with either irregular curvilinear crooked shape or straight line shape with plurality of peaks and valleys across its axial length. The closed cells (9) are formed by connecting valleys of elements in upper row (upper ring of elements) with the peaks of the elements in the lower row (lower ring of elements) across the axis of the stent when the stent is viewed in vertical position. Connection of peaks and valleys of two rows of elements along the axis of the stent forms one row of closed cells. Additional row of closed cells can be formed by connecting peaks and valleys of three consecutive rows of elements along the axis of the stent and so on. The open cells (12) are formed by using 's' shaped links to connect upper and lower rows anywhere along length of the sides of the elements without interconnecting the peaks and valleys of these sinusoidal elements anywhere along length of their sides. The closed cells and open cells with 's' shaped linkages are interconnected to form cylindrical scaffold structure of the stent.

The closed cells at the end sections of the stent have higher strength i.e. they offer higher resistance to expansion. The 's' shaped linkages provide flexibility to the stent for easy maneuvering of the stent in curved and tortuous paths of the body lumen. The structural strength of the irregular curvilinear line element can be changed by changing the location where 's' shaped interconnections are attached along the length of the element. In the embodiments described in this invention, 's' shaped linkages are located nearly at the center of respective sides of the elements. The width and shape of individual strut is designed to provide effective crimping, to impart sufficient radial strength in expanded state and at the same time to keep recoil and foreshortening within acceptable limits. The scaffold structure after expansion gives acceptable crossing profile. The irregular curvilinear line structure has varying degrees of curvature in regions of the peaks and valleys. The curvature can be varied to impart different structural strength. Its shape should give uniform and low crimping profile as well as uniform radial expansion of individual elements around the circumference of the stent in a section and in individual layers along the axis of the stent. When rated deployment pressure is applied to the stent through the balloon of the catheter, the stent attains a uniform diameter across its entire length in spite of having differential strength axially.

The sinusoidal scaffold structures are designed with struts and 's' shaped connecting linkages to give segments which are highly flexible. On expansion of the stent during its deployment, these segments deform circumferential ly from crimped diameter to an enlarged expanded diameter. Different radial expansion characteristics can be obtained by changing size, shape and cross-section of the sinusoidal element and 's' linkage structures. In addition, the strength (resistance to expansion) of the end sections of the stent can be increased by increasing the number of rows of the closed cells or also by changing the number of cells in a row. Similarly, the strength (resistance to expansion) of the open cells can be increased by increasing the number and width of 's' shaped linkages or by changing number of open cells in a row. The location where the 's' shaped linkages connect the upper and lower rows of open cells can also be manipulated to increase or reduce the strength of open cells and the overall flexibility of the stent.

The shape of the open and closed cells can be changed by changing the curvature of their sides. In a limit, they can be given shape of a straight line. Such changes can make a difference in overall strength of the cell and hence the strength of the row and stent structure as a whole.

The geometry of the interconnected scaffold structure of the stent is so designed that the elastic recoil and foreshortening of the stent on expansion are kept within acceptable limits.

The scaffold structure of the stent of one embodiment of the present invention consists of three rows of elements forming closed cells each at the distal end and the proximal end of the stent. The valleys of the upper row of elements are connected to the peaks of the lower row of elements to form a honeycomb like web of interconnected arrays of closed cells. The central section starts where the closed cell structure ends. This section consists of open cells. The number of rows of elements to from open cells in the central section is dictated by overall length of the stent. For example, for stent of one specific configuration with 13 mm overall length, there are 5 rows of open cells interconnected alternately with 's" shaped linkages as described above. Stent of the same configuration with 38 mm ength has 23 - 24 such rows. The number of cells in a row along the circumference of he stent, defined as crowns, is dictated by the diameter of the stent and width of the cell. For example, a stent of this configuration with 2.5 mm diameter may have 3 crowns across its circumference while a stent of 4.5 mm diameter of same configuration has 5 crowns across its circumference. The number of crowns can be changed keeping balance with crimping profile.

The overall configuration of the stent controls the radial expansion with central section expanding earlier than the end sections because the end sections are more resistant to expansion process. This configuration also decides the radial strength, flexibility and fatigue resistance of the stent. The dimensions of each cell and their spacing are adjusted close enough to prevent protrusion of the plaque or any part of the body lumen where the stent is implanted. At the same time, these dimensions are adjusted to achieve trouble free crimping of the stent over the balloon of the catheter without compromising the flexibility of the stent. The spacing is also adjusted to give desired crossing profile. This configuration gives uniform coverage of lumen wall with the stent struts after the stent is fully expanded. The stent gets nicely and firmly apposed in the body lumen. During deployment, the individual elements of sections may be disturbed slightly relative to adjacent cylindrical elements without deforming the overall scaffold structure. After the stent is expanded, portions of the elements may slightly tip outwardly and embed in the vessel wall a little to position the stent properly in the body lumen. This aids in apposing the stent firmly in place after expansion.

The configuration of the individual cells, the "s" linkages and their interconnections are designed to distribute the stresses during crimping and expansion uniformly across the entire stent. This allows the central section of the stent to expand earlier than the end sections and stent achieves uniform diameter after it is fully expanded.

The interconnection of open cells with each other is achieved by the "s" shaped linkages as described above. These linkages are connected nearly at the center of the side of the element forming the sinusoidal wave type shape of a cell. This gives a structure which is in the form of a well supported structural beam in which the unsupported length is reduced at the connection point of 's' linkage like a cross linked truss girder beam. The "s" shaped linkages can also be connected off-center to the side elements of the open cells. This will divide the unsupported length of this element in 3 sections. The unsupported length of these elements depend on the positions of these "s" shaped linkages. This gives additional strength to the overall structure of the stent and it resists foreshortening. The elements of the cells undergo full plastic deformation after expansion to keep elastic recoil well within acceptable limits.

The configuration of the stent scaffold structure gives enough leeway to a stent designer to vary the shapes and other dimensions of the elements of the stent to effectively reduce the thickness of the stent struts keeping the radial strength within acceptable limits and get desired fatigue resistance. It is a well accepted fact that reduced thickness of the stent reduces injury to the walls of the body lumen.

Flexibility of the stent is decided by the thickness and number of "s" shaped linkages across the circumference of the stent as well as their locations. If the number of these "s" connectors is reduced, some of the sinusoidal sections become free to give more flexibility to the stent. However, this will reduce the radial strength of the stent. Thus it is extremely important to strike a balance between the flexibility and the strength to optimize overall properties of the stent.

The designs of stents described in this invention are generally for coronary vasculature.

However, the configurations described allow varying the shapes and other dimensions of the elements of the stent such that it is possible to make stents for other applications like cerebral vasculature, renal vasculature etc. For example, it is possible to reduce the radial strength and increase the flexibility of stent by changing the configuration to get properties desirable for cerebral vasculature. By providing closed cell configuration only at one end of the stent it is possible to make it suitable for some special application. If no closed cells are provided, the differential expansion of the stent is eliminated to make it suitable for renal application. In this way, the stent structure configuration described in this invention gives enough flexibility to a stent designer to tailor the stent for any application.

### BRIEF DESCRIPTION OF THE DRAWINGS:

Fig. 1 is a view depicting the stent mounted on a delivery catheter 1.
Fig 2 depicts an enlarged view of the stent 3 of Fig 1 crimped on the balloon 2 of the delivery catheter 1.
Fig. 3 depicts the stent of Fig. 1 during and after its expansion in the body lumen schematically; Fig 3A depicts the stent 3 being expanded within a body lumen having a plaque 4. Fig 3B depicts the same stent 3 schematically after its full expansion is achieved and the catheter is withdrawn after deflation from the body lumen. This figure does not depict real implantation where the struts of the stent penetrate the plaque 4 and body lumen 5.
Fig. 4 depicts the preferred embodiment of the stent 3 showing sinusoidal elements (9', 9" and 12' and 12") and "s" shaped interconnecting links 13 as arranged across the body of the stent with end sections (6 and 7) and central section 8. It also depicts closed cell 9 made of elements 9', 9" and two similar elements of the lower row. It depicts the open cell 12 made by elements 12'and 12". In this figure, the shapes of elements 9', 9", 12' and 12" are different. These shapes may be same or different to achieve different structural strengths and other properties.
Fig. 5 depicts an enlarged view of typical sinusoidal elements 9' and 9" forming closed cell 9 which is a part of the end section of the stent.
Fig. 6 depicts an enlarged view of typical sinusoidal open element 12' and 12" forming open cell 12 with "s" shaped interconnecting link 13 connected nearly at the center of the length of element 12'. In this embodiment, the "s" shaped linkages are connected to open cells intermittently. These links may be connected to each open cell to achieve different structural strengths and other properties.
Fig. 7 depicts an enlarged view of the sinusoidal elements 12' and 12" forming open cell of Fig 5.
Fig 8 depicts enlarged view of a typical "s" shaped interconnecting link 13 of Fig 6.
Fig 9 depicts pictures taken during gradual and controlled expansion of the stent. These pictures clearly demonstrate the characteristic of the stent to start expanding from the central section earlier than the end sections.
Fig 10 depicts one arrangement of closed cells in the end section of the stent where all cells are interconnected.
Fig 11 depicts an arrangement alternate to that of Fig 10 where closed cells in the end section of the stent are interconnected alternately.
Fig 12 depicts an arrangement where the "s" shaped interconnecting links are connected to alternate open cells.
Fig 13 depicts an arrangement where the "s" shaped interconnecting links are connected to all the sinusoidal elements of the open cells.
Fig 14 depicts a typical coronary stent configuration which has average strut thickness of 65 microns.
Fig 15 depicts a typical coronary stent configuration which has average strut thickness of 35 microns.
Fig 16 depicts a renal stent configuration which has average strut thickness of 50 microns.
Fig 17 depicts a stent configuration with closed cells only at one end.
Fig 18 depicts a stent configuration with all open cells and no closed cells.

### DETAILED DESCRIPTION OF THE PREFERRED EMBODIMENTS:

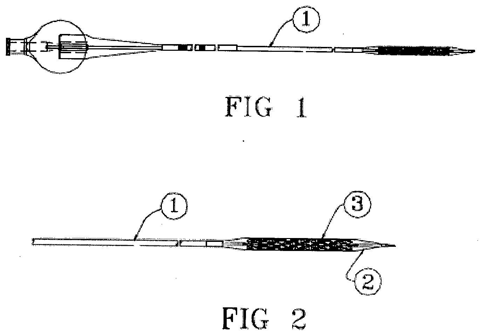

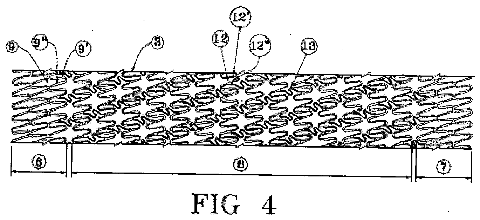

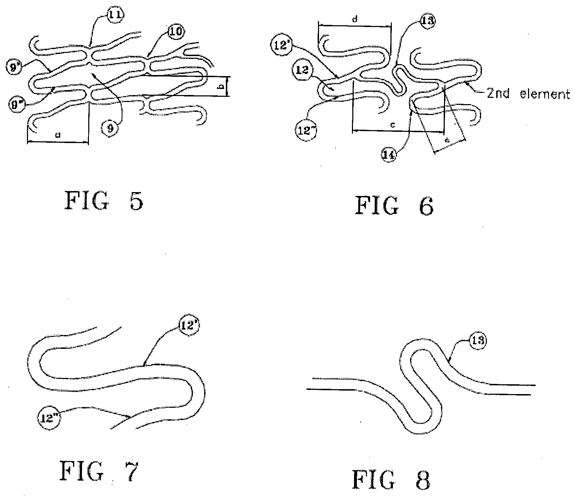

As shown in Figs. 1 through 4, the preferred embodiment of the present invention includes stent 3 consisting of central section 8 and identical end sections 6 and 7 along the longitudinal axis of the stent. All the sections have expandable strut elements 9', 9", 12', 12" and 13 having a plurality of sinusoidal wave shaped sections 9 (closed) and 12 (open) linked in specific manner by direct connections or through "s" shaped linkages to form entire body of the stent. The sinusoidal shaped closed cells 9 have struts 9' and 9" as shown in Fig 5. The sinusoidal shaped open cells 12 have struts 12' and 12" as shown in Fig 6. The struts 9' and 9" of closed cells 9 are interconnected with each other across their length forming a joint 11 and at their tips forming a joint 10 as shown in Fig 5. The open cell 12 in one row is joined to another open cell 14 in the next row by "s" shaped interconnecting link 13 as shown in Fig 6. The width of the struts 9', 9" 12', 12" and 13 may be same or different and can be adjusted to achieve desired properties of the stent. The shape of these elements and cells and the widths of the struts can be adjusted such as to impart desirable mechanical strength and flexibility to the stent and also to ease the process of crimping. The stent expands uniformly across any axial cross section and achieves specified diameter across its entire length at specified deployment pressure.

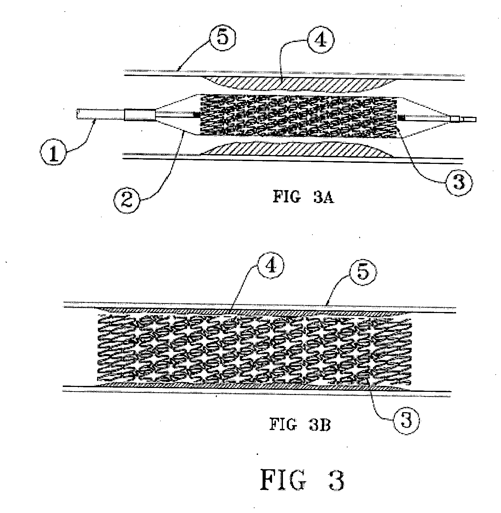

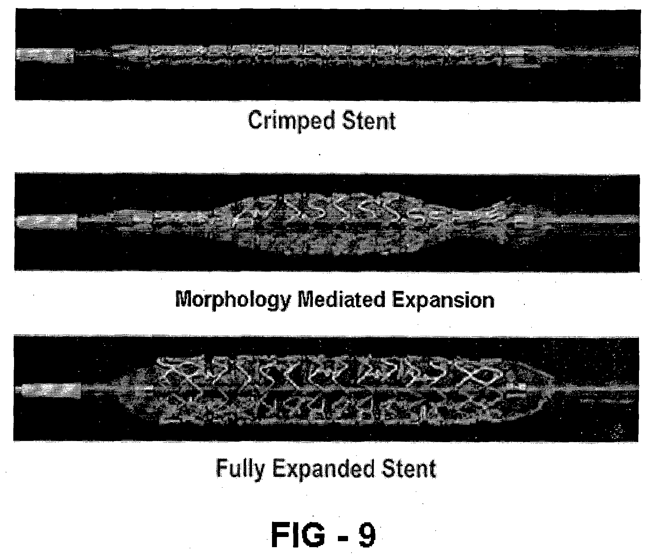

During deployment of the stent, the balloon of the delivery catheter is expanded by applying hydraulic pressure. This exerts force on the stent which is crimped on the balloon causing it also to expand radially outwards along with the balloon 2 (Fig 2 and 3A). The struts of the cells (9', 9", 12', 12" and 13) experience tensile force. This force causes these elements to deform in their respective shapes in specific manner to cause uniform expansion of the stent across its circumference at a specific cross section along the axis of the stent. Due to different mechanical strengths (i.e. different resistance to radial expansion) of the central and end sections of the stent, the degree of expansion of different sections is different. The end sections are made up of closed cells which have higher mechanical strength compared to the central section which is made up of open cells which have relatively lower mechanical strength. This differential strength causes the central section to expand earlier than the end sections as evident from Fig 9. When the balloon reaches the specified deployment pressure, the stent attains specified diameter across its entire length.

Size, shape, width, thickness and cross supporting of the sinusoidal cell structure may be varied to achieve different mechanical strength of a section of the stent which in turn can produce different expansion characteristics. As described above, closed cells are more resistant to expansion forces than the open cells connected by "s" shaped interconnecting links.

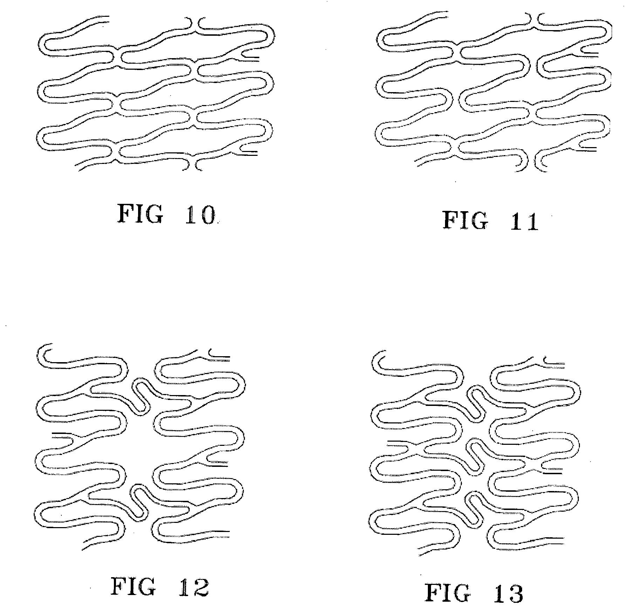

In one embodiment, the end sections are made of closed cells which are all interconnected with each other as shown in Fig 10.

In another embodiment, not according to the present invention, the end sections are made of closed cells which are alternately interconnected as shown in Fig 11. It is obvious that the arrangement as depicted in Fig 10 will result in higher mechanical strength of this section than the one with arrangement as depicted in Fig 11. Similarly, the strength of the central section can be varied by altering the arrangement of interconnecting "s" shaped links as depicted in Fig 12 and 13. The "s" shaped links can be provided on alternate sinusoidal cells as depicted in Fig 12 or on all sinusoidal cells as depicted in Fig 13. Obviously the arrangement of Fig 13 will give more strength than that of Fig 12. In addition, the strength of any section or cell can be increased by increasing the width of the strut or increasing the thickness of the strut. The latter will have more profound effect on the strength. Thus, these arrangements offer a vast opportunity to the designer in manipulating the geometry of scaffold of the stent to achieve desired relative strengths in various sections of the stent as well as flexibility of the stent.

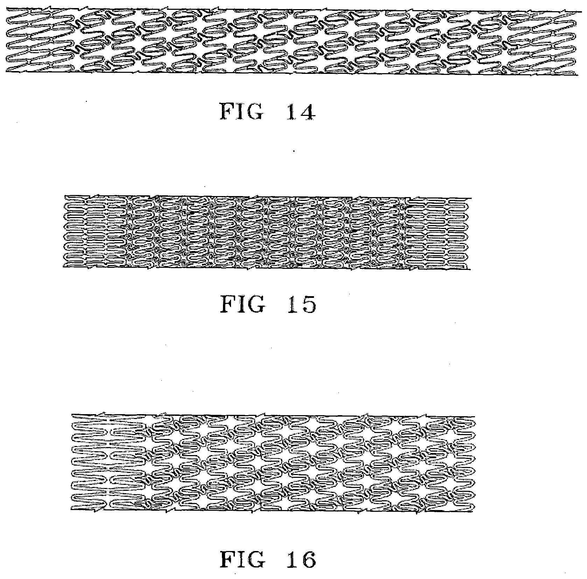

Changing the shape of the elements forming a cell can be used to alter the mechanical strength of the cell. Figs. 14, 15 and 16 depict different shapes of the elements forming closed cells at the ends of the stents. Fig 14 shows elements having sinusoidal curvilinear shape. The shapes of two adjacent elements are not same. Fig 15 and 16 show the elements made of straight line shape with arcs at the end. The closed cells of Fig 15 have parallel elements while those of Fig 16 are not parallel. The relative strengths and flexibility of each of these arrangements are different. These figures give an idea of the possibilities of changing the shapes and sizes to achieve different strengths and other properties.

The strength of a cell can be increased by reducing its dimensions. For example, refer to Fig 5 of the closed cell. The strength of the cell can be increased by reducing the dimension 'a' and 'b'. Similarly the strength of the open cell can be increased by reducing the dimension 'd' (refer to Fig 6) and vice versa. The strength of a section in the central section can be increased by reducing the dimension 'c' (refer to Fig 6). The location of connecting the "s" shaped linkage 13 to the element 12' or 12" (dimension e) will also change the strength (refer to Fig 6). Care should be taken not to make the crimping and expansion difficult or affecting the flexibility of the stent adversely while adjusting these dimensions.

All the aspects mentioned above offer a vast opportunity to a stent designer to vary the properties of the stent. The dimensions and shapes of the cells can be adjusted to achieve a scaffold structure of the stent which results into optimum mechanical properties to achieve better clinical performance.

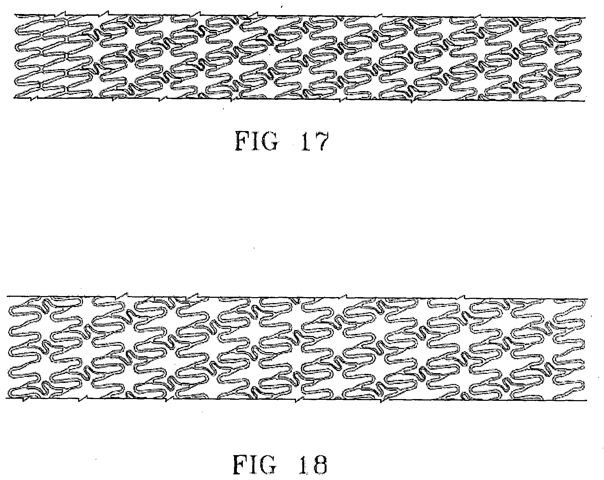

In another embodiment, not according to the invention, one end of the stent is made of closed cells and the rest of the portion of the stent has open cells as depicted in Fig 17. In this case, the end having open cells will expand first followed by expansion of the rest of the stent. The other end having closed cells will expend last. This feature has the ability to allow for better conformability of the cells across a variety of lesion morphologies and also propensity to minimize edge injury.

In yet another embodiment, not according to the invention, the entire stent is made of open cells and "s" shaped inter connecting links as shown in Fig 18. This stent will exhibit dog boning effect. Such configurations are preferred in applications like implantation in cerebral vasculature as 's' shaped linkages provide high flexibility. The strut thickness in this case can be low to make the stent expand at a lower pressure. Such stents can also be used for below the knee implantations. However, for this application, the strut thickness may be increased to give more radial strength and fatigue resistance. Thus the invented structural configuration of the stent offers innumerable preferred possibilities which can be tailored to the application.

This invention describes the stent structure that does not require differential heat treatment or differential electropolishing processes. Hence the thickness of struts remains constant across the entire axial length and circumference of the stent. Thus, the thickness of struts is also the thickness of the entire stent.

All aspects described above are illustrated in following preferred embodiments of coronary stents.

The coronary stent is made from Cobalt Chromium alloy L-605 preferably by methods well known in the art of making coronary stent. The tube used for making the stent should be thin walled and with accurate dimensions. The manufacturing steps are described below.
1. Cutting of the tube on accurate Laser cutting machine having a thin laser beam to get precise scaffold geometry.
2. Cleaning and descaling of the cut stent using standard methods.
3. Heat treating the descaled stent to get desired microstructure as well as mechanical strength and fatigue resistance of the metal.
4. Electropolishing the heat treated stent to achieve desired surface properties and precise final dimensions of the struts (widths and thickness) by accurately controlling the process parameters.
5. Crimping the stent on the balloon of catheter as such or after coating with therapeutic agents/biocompatible materials.

In this preferred embodiment of a coronary stent, the configuration of the stent and its overall scaffold structure are shown in Fig 14. For various sizes of the stent, the configuration of the end sections is identical i.e. the number of closed cells across the longitudinal axis of the stent is same for all sizes. The number of open cells in the central section across the longitudinal axis of the stent is varied to achieve desired overall length of the stent. Number of closed or open cells across the circumference of the stent is different for different diameters of the stent. The thickness of struts in this preferred embodiment was average 65 microns which is thinner compared to other stents of comparable sizes.

The stents of various sizes were subjected to various mechanical tests the results of which are given below.

| **Property** | **Description of Test & Test Articles** |
|---|---|
| Foreshortening | Foreshortening was examined as per EN 14299: 2004. The mean foreshortening value was between 0.29% and 0.41% at Rated |
| | Burst Pressure of the balloon, which were below (better) the value of predicate devices tested. |
| Uniformity of Expansion (Dogboning Examination) | The dilation behavior of the crimped stents was examined as per EN 14299: 2004 and none of the stents showed dogboning effect. The stents expanded from the central portion first. The stents maintained uniformity upon withdrawal of the balloon catheter. |
| Elastic Recoil | The dilation behavior of stents at the labeled diameter and after recoil was examined as per EN 14299:2004. The mean value of recoil was between 3.38% and 3.49%, which was within the range of the value of predicate devices tested. |
| Radial strength | Radial strength of the stent was determined as per EN 14299:2004. The mean collapse pressure was between 1.7 bar and 1.1 bar, which was above (better) the mean value reached by the predicate devices. |
| Stent Fatigue Test | The accelerated fatigue test was performed according to EN 14299:2004 to 400 million cycles. The stents successfully completed 400 million cycles. |
| Trackability | Trackability of stent systems was examined according to EN 14299:2004. Ultimate force required for various stent sizes were between 3.24N and 2.89N. The mean ultimate force values were below the mean value (better) of all predicate devices tested. |
| Pushability | Pushability of the delivery system was examined according to EN 14299:2004. The mean ultimate force value for various stent sizes was between 0.39N and 0.73N, which was below (better) those of all predicate devices tested. |

In another preferred embodiment, a coronary stent was made with struts thinner than the preferred embodiment described above. The stent is made from Cobalt Chromium ally L-605 by methods described in embodiment above. In this preferred embodiment, the configuration of the stent and its overall scaffold structure are shown in Fig 15. For various sizes of the stent, the configuration of the end sections is identical i.e. the number of closed cells across the longitudinal axis of the stent is same for all sizes. The number of open cells in the central section across the longitudinal axis of the stent is varied to achieve desired overall length of the stent. Number of closed or open cells across the circumference of the stent is different for different diameters of the stent. The thickness of struts in this prepared embodiment was average 35 microns which is much thinner compared to any other stents of comparable sizes available in the market.

The stents of various sizes of above embodiment were subjected to various mechanical tests the results of which are given below.

| **Test** | **Description of Test & Test Articles** |
|---|---|
| Foreshortening | Foreshortening was examined as per EN 14299: 2004. Mean foreshortening value was found between 0.49% and 0.78% at Rated Burst Pressure, which were below (better) the value of predicate devices tested. |
| Uniformity of Expansion (Dogboning Examination) | The dilation behavior of the crimped stents was examined during the inflation as per EN 14299: 2004. None of the stents showed dogboning effect. Stents of various sizes maintained uniformity upon withdrawal of the balloon catheter. |
| Elastic Recoil | The dilation behavior of coronary stents at its labeled diameter and after recoil was examined as per EN 14299:2004. The mean value of recoil was between 3.47% and 3.69%, which was within the range of the value of predicate devices tested. |
| Determination of radial strength | Radial strength was determined as per EN 14299:2004. The mean collapse pressure was between 1.5 bar and 1.2 bar, which was above (better) the mean value reached by the predicate devices tested. |
| Stent Fatigue Test | The accelerated fatigue test was performed according to EN 14299:2004 to 400 million cycles. The stents successfully completed 400 million cycles. |
| Trackability | Trackability of stent systems was examined according to EN 14299:2004. Ultimate force required for various stent sizes were between 2.8N and 1.6N. The mean ultimate force values were below the mean value (better) of all predicate devices tested. |
| Pushability | Pushability of the delivery system was examined according to EN 14299:2004. The mean force required to push the deliver system was between 0.22N and 0.68N, which were below (better) all predicate devices tested. |

In another preferred embodiment, a renal stent is made from Cobalt Chromium alloy L-605 using same overall configuration of the invention. The stent is made from Cobalt Chromium ally L-605 by methods described in embodiments above. In this preferred embodiment, not according to the present invention, the configuration of the stent and its overall scaffold structure are shown in Fig 16. For various sizes of the stent, the configuration of the end sections is identical i.e. the number of closed cells across the longitudinal axis of the stent is same for all sizes. The number of open cells in the central section across the longitudinal axis of the stent is varied to achieve desired overall length of the stent. Number of closed or open cells across the circumference of the stent is different for different diameters of the stent. The thickness of struts in this preferred embodiment was average 50 microns which is intermediate to the two embodiments described above.

The stents of various sizes of above embodiment were subjected to various mechanical tests the results of which are given below.

| **Test** | **Description of Test & Test Articles** |
|---|---|
| Foreshortening | Foreshortening of various stents sizes was examined as per EN 14299: 2004. The mean foreshortening value was between 0.32% and 0.45% at Rated Burst Pressure, which were below (better) the value of predicate devices tested. |
| Uniformity of Expansion (Dogboning Examination) | The dilation behavior of the crimped stents was examined during the inflation as per EN 14299: 2004 and none of the stents showed dogboning effect. The various stent sizes maintained uniformity upon withdrawal of the balloon catheter. |
| Elastic Recoil | The dilation behavior of coronary stents at its labeled diameter and after recoil was examined as per EN 14299:2004. The mean value of recoil was between 3.42% and 3.57%, which was within the range of the value of predicate devices tested. |
| Radial strength | Radial strength of the stent was determined as per EN 14299:2004. The mean collapse pressure was between 1.45 bar and 1.2 bar, which were above (better) the mean value reached by the predicate devices tested. |
| Stent Fatigue Test | The accelerated fatigue test was performed according to EN 14299:2004 to 400 million cycles. The stents successfully completed 400 million cycles accelerated fatigue without fatigue failure. |
| Trackability | Trackability of mounted stent systems was examined according to EN 14299:2004. Ultimate force required for various stent sizes was between 3.02N and 2.14N, which was below the mean value (better) of all predicate devices tested. |
| Pushability | Pushability of the delivery system was examined according to EN 14299:2004 The mean ultimate force for various stent sizes was found between 0.3N and 0.62N, which was below (better) the all predicate devices tested. |

As evident from the above embodiments, the scaffold structure can be altered in a way that results into desired mechanical strength with varying thickness of the stent.

The embodiment describing the stent with 35 micron strut thickness is of specific significance. It is well established that lower the strut thickness, lower is the injury to the blood vessel. Lower injury results into lower restenosis of the vessel and less post implantation clinical complications. The stent with 35 micron strut thickness has adequate mechanical strength and other desirable properties. The metal to artery ratio in this embodiment is high enough to allow required drug loading with lower coating thickness. The crossing profile for the stent of this embodiment is 0.98 mm (for 3.0mm balloon dia) which is adequate.

Radiopacity of the stent with 35 microns thickness was comparable to other predicate stents. X-ray images of 35 microns thick stent (Mitsu) along with those of Driver^{®} and Vison^{®} are given below.

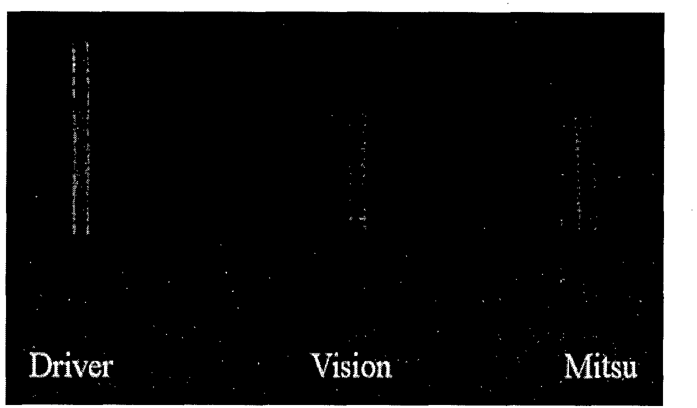

The thickness of the stent can be reduced to less than 35 microns by using a metal or alloy which is mechanically stronger than Cobal-Chromium alloy L-605 and has adequate radiopacity for making the stent.

### Advantages:

1. The configuration of closed and open cells to give different mechanical strengths.
2. Stent where central section expands first - no dog boning effect.
3. The shape that can give structural flexibility by change in shapes, dimensions and attachment numbers and locations.
4. Stent with thinnest struts having adequate mechanical properties.

## Claims

1. A balloon expandable metallic stent (3) for implantation in body lumen having a scaffold structure of uniform strut thickness as low as 35 microns comprising sinusoidal wave type elements with either irregular curvilinear crooked shape or straight line shape with plurality of peaks and valleys
a) wherein the stent comprises: two end sections (6, 7) wherein both the end sections comprise multiple rows of closed cells (9) formed by connecting all the valleys of sinusoidal elements (12', 12") in an upper row with corresponding peaks of sinusoidal elements in a lower row across the axis of the stent; and
b) the central section (8) of the stent scaffold comprising multiple rows of open cells (12) formed by connecting upper and lower rows of sinusoidal elements (9', 9", 12', 12") with 's' shaped links (13) anywhere along the length of the sides of the elements without interconnecting the peaks and valleys of these elements,
wherein the open cells are interconnected to each other with 's' type linkages (13) anywhere along the length of the sides of the elements without interconnecting the peaks and valleys of these elements throughout the entire length of the stent,
wherein, the distinct end and central sections have varying geometry along the axial length of the stent and impart varying resistance to radial expansion to these sections; the closed cells being mechanically stronger thereby offering more resistance to radial expansion than the open cells;
wherein the stent is made of Cobalt Chromium alloy L-605; and
wherein the open cells and closed cells of adjacent rows along the axis of the stent are interconnected with each other with 's' type linkages (13) anywhere along the length of the sides of the elements without interconnecting the peaks and valleys of these elements throughout the entire length of the stent.

2. The balloon expandable stent according to Claim 1 wherein the "s" shaped interconnecting linkages impart flexibility to the stent structure.

3. The balloon expandable stent according to any one of the preceding Claims, wherein differential mechanical strength is imparted to different sections of the stent across its axial length without requiring differential heat treatment, use of different materials and differential electropolishing treatment, and wherein the thickness of struts is maintained along the entire axial length of the stent making the thickness of stent uniform along its entire structure.

## Patentansprüche

1. Ballonexpandierbarer Metallstent (3) zur Implantation in einem Körperlumen, der eine Gerüststruktur mit einheitlicher geringer Strebendicke bis herunter auf 35 Mikrometer aufweist, umfassend sinuswellenartige Elemente mit entweder unregelmäßig krummliniger gekrümmter Form oder geradliniger Form mit einer Vielzahl von Spitzen und Tälern,
a) wobei der Stent Folgendes umfasst: zwei Endabschnitte (6, 7), wobei beide Endabschnitte mehrere Reihen geschlossener Zellen (9) umfassen, die durch Verbinden aller Täler sinusförmiger Elemente (12', 12") in einer oberen Reihe mit entsprechenden Spitzen sinusförmiger Elemente in einer unteren Reihe über die Achse des Stents hinweg gebildet sind; und
b) wobei der mittlere Abschnitt (8) des Stentgerüsts mehrere Reihen offener Zellen (12) umfasst, die durch Verbinden oberer und unterer Reihen sinusförmiger Elemente (9', 9", 12', 12") mit "s"-förmigen Verbindungsgliedern (13) an beliebiger Stelle entlang der Länge der Seiten der Elemente, ohne die Spitzen und Täler dieser Elemente miteinander zu verbinden, gebildet sind,
wobei die offenen Zellen mit "s"-förmigen Verbindungsgliedern (13) an beliebiger Stelle entlang der Länge der Seiten der Elemente, ohne die Spitzen und Täler dieser Elemente miteinander zu verbinden, auf der gesamten Länge des Stents miteinander verbunden sind,
wobei die Endabschnitte und der mittlere Abschnitt, die ungleich sind, eine veränderliche Geometrie entlang der Axiallänge des Stents aufweisen und diesen Abschnitten einen veränderlichen Widerstand gegen radiale Expansion verleihen; wobei die geschlossenen Zellen mechanisch fester sind und dadurch mehr Widerstand gegen radiale Expansion bieten als die offenen Zellen;
wobei der Stent aus Kobalt-Chrom-Legierung L-605 hergestellt ist; und
wobei die offenen Zellen und die geschlossenen Zellen benachbarter Reihen entlang der Achse des Stents mit "s"-förmigen Verbindungsgliedern (13) an beliebiger Stelle entlang der Länge der Seiten der Elemente, ohne die Spitzen und Täler dieser Elemente miteinander zu verbinden, auf der gesamten Länge des Stents miteinander verbunden sind.

2. Ballonexpandierbarer Stent nach Anspruch 1, wobei die "s"-förmigen Verbindungsglieder der Stentstruktur Biegsamkeit verleihen.

3. Ballonexpandierbarer Stent nach einem der vorangehenden Ansprüche, wobei verschiedenen Abschnitten des Stents über seine axiale Länge hinweg unterschiedliche mechanische Festigkeit verliehen wird, ohne unterschiedliche Wärmebehandlung, die Verwendung verschiedener Materialien und unterschiedliche Elektropolierbehandlung zu benötigen, und wobei die Dicke von Streben entlang der gesamten axialen Länge des Stents beibehalten wird, sodass die Dicke des Stents entlang seiner gesamten Struktur einheitlich ist.

## Revendications

1. Endoprothèse métallique déployable à ballonnet (3) destinée à être implantée dans une lumière corporelle ayant une structure d'échafaudage présentant une épaisseur d'entretoise uniforme aussi faible que 35 microns comprenant des éléments de type onde sinusoïdale avec soit une forme courbée curvilinéaire irrégulière ou une forme en ligne droite avec une pluralité de crêtes et de vallées
a) dans laquelle l'endoprothèse comprend : deux sections d'extrémité (6, 7) dans laquelle les deux sections d'extrémité comprennent plusieurs rangs de cellules fermées (9) formées par connexion de toutes les vallées d'éléments sinusoïdaux (12', 12") dans un rang supérieur avec les crêtes correspondantes d'éléments sinusoïdaux dans un rang inférieur sur l'axe de l'endoprothèse ; et
b) la section centrale (8) de l'échafaudage d'endoprothèse comprenant plusieurs rangs de cellules ouvertes (12) formées par connexion de rangs supérieurs et inférieurs d'éléments sinusoïdaux (9', 9", 12', 12") avec des liaisons en forme de « s » (13) n'importe où le long des côtés des éléments sans interconnexion des crêtes et des vallées de ces éléments,
dans laquelle les cellules ouvertes sont interconnectées les unes avec les autres avec des liaisons en « s » (13) n'importe où le long des côtés des éléments sans interconnexion des crêtes et des vallées de ces éléments sur toute la longueur de l'endoprothèse,
dans laquelle les sections d'extrémité et centrale distinctes ont une géométrie variable sur la longueur axiale de l'endoprothèse et communiquent une résistance variable au déploiement radial de ces sections ; les cellules fermées étant plus fortes mécaniquement offrant ainsi plus de résistance au déploiement radial que les cellules ouvertes ;
dans laquelle l'endoprothèse est fabriquée dans un alliage cobalt-chrome L-605 ; et
dans laquelle les cellules ouvertes et les cellules fermées de rangs adjacents le long de l'axe de l'endoprothèse sont interconnectées les unes avec les autres avec des liaisons en « s » (13) n'importe où le long des côtés des éléments sans interconnexion des crêtes et des vallées de ces éléments sur toute la longueur de l'endoprothèse.

2. Endoprothèse déployable à ballonnet selon la revendication 1, dans laquelle les liaisons d'interconnexion en « s » communiquent une flexibilité à la structure d'endoprothèse.

3. Endoprothèse déployable à ballonnet selon l'une quelconque des revendications précédentes, dans laquelle une force mécanique différentielle est communiquée à des sections différentes de l'endoprothèse sur sa longueur axiale sans nécessiter de traitement thermique différentiel, l'utilisation de matériaux différents et de traitement d'électropolissage différentiel, et dans laquelle l'épaisseur des entretoises est maintenue sur toute la longueur axiale de l'endoprothèse rendant l'épaisseur d'entretoise uniforme sur toute la longueur de sa structure.
